# EUROPÄISCHE PATENTSCHRIFT

(11) **EP 2 405 844 B1**
(45) Veröffentlichungstag und Bekanntmachung des Hinweises auf die Patenterteilung: **27.02.2019**
(21) Anmeldenummer: 10709412.0
(22) Anmeldetag: 10.03.2010
(51) Int. Cl.: A61B 18/14, A61B 18/00, A61B 17/00

(54) **SCHALTSTEUEREINRICHTUNG UND HANDHABUNGSTEIL EINES MEDIZINISCHEN INSTRUMENTS**
SWITCHING CONTROL DEVICE AND MANIPULATING PART FOR A MEDICAL INSTRUMENT
DISPOSITIF DE COMMANDE DE COMMUTATION ET MANCHE D'UN INSTRUMENT MÉDICAL

(30) Priorität: 12.03.2009 DE 102009012910; 05.03.2010 DE 102010000642
(43) Veröffentlichungstag der Anmeldung: 18.01.2012
(73) Patentinhaber: Erbe Elektromedizin GmbH, 72072 Tübingen (DE)
(72) Erfinder: SCHNITZLER, Uwe, 72074 Tübingen (DE)
(74) Vertreter: Bohnenberger, Johannes
(86) Internationale Anmeldenummer: PCT/EP2010/001490
(87) Internationale Veröffentlichungsnummer: WO 2010/102800

(56) Entgegenhaltungen:
- EP-A2- 1 199 040
- WO-A2-2008/152378
- DE-A1- 2 460 481
- DE-A1- 19 854 313

## Beschreibung

Die Erfindung betrifft eine Schaltsteuereinrichtung eines medizinischen Instruments, insbesondere einer Elektrode für die HF-Chirurgie, sowie ein Handhabungsteil eines solchen Instruments.

Mit dem Begriff "Schaltsteuereinrichtung" werden hier beliebige Schalter und/oder Steuereinrichtungen bezeichnet, mit denen ein Bediener eine Funktion des betreffenden Instruments, insbesondere eine Energiezufuhr zu diesem, eine geometrische Konfiguration desselben oder eine Signalübermittlung von diesem, schalten oder in sonstiger Weise (insbesondere auch analog) steuern kann. Unter einem "Instrument" wird grundsätzlich jedes ärztliche - chirurgische, therapeutische oder diagnostische - Instrument verstanden, und als "Handhabungsteil" werden Teile oder Abschnitte eines solchen Instruments bezeichnet, die ein Bediener in der Hand hält und/oder an denen er Schalt- bzw. Steuerfunktionen beim ärztlichen Gebrauch des Instruments ausführt. Es werden ausdrücklich auch endoskopische Instrumente, die kein Handhabungsteil im engeren Sinne aufweisen, aber von einem Bediener geführt und geschaltet/gesteuert werden, als zum Bereich der Erfindung gehörig angesehen.

Derzeit auf dem Markt befindliche wiederverwendbare chirurgische Handhabungselemente mit Aktivierungsfunktion (z.B. Elektrodengriffe für die HF-Chirurgie) werden derart ausgeführt, dass die Schaltfunktion bzw. die hierfür notwendigen elektronischen Bauteile wie z.B. Taster, Widerstände, Dioden, auf einer Leiterplatte angebracht sind, welche zentral im Inneren der Griffe integriert ist.

Die elektrischen Kabelanschlüsse (Litzen) werden in aller Regel auf der Leiterplatte angelötet. Die zur elektrischen und mechanischen Kontaktierung der Instrumente (z.B. monopolarer Elektroden) notwendige Kontaktbuchse wird ebenso auf der Leiterplatte (z.B. durch Lötverfahren) befestigt. Bei HF-Chirurgiehandgriffen wird die zur Anwendung notwendige Leistung somit über die auf der Leiterplatte angebrachten Leiterbahnen geleitet.

Chirurgische Handhabungselemente mit Aktivierungsfunktion (z.B. Elektrodengriffe für die HF-Chirurgie, wie z.B. beschrieben in der Offenlegungsschrift EP 1 199 040) werden durch Sterilisation mittels feuchter Hitze, Ethylenoxid-Gas oder Gamma-Bestrahlung aufbereitet. Es muss sichergestellt werden, dass die Produkte über einen festgelegten Zeitraum ohne Einschränkung ihre Funktion erfüllen und dabei keinerlei sicherheitsrelevante Mängel entstehen, welche zu einer Gefährdung von Patient und Anwender führen. Die Umgebungsbedingungen (thermisch, physikalisch) der unterschiedlichen Sterilisationsverfahren, können zum vorzeitigen Ausfall der Produkte, z.B. durch Eindringen von Flüssigkeiten, führen.

Für die Aufrechterhaltung der einwandfreien Funktion und den Ausschluss sicherheitsrelevanter Mängel ist es notwendig, das Eindringen von Feuchtigkeit (initiiert durch Aufbereitung: Waschen, Sterilisieren) zu verhindern. Dies geschieht in aller Regel durch mechanische Abdichtung (z.B. O-Ringe) an den Schnittstellen zur äußeren Umgebung, z.B. Kabelaustritt, Tastenbereich, Kontaktbuchsenbereich am distalen Griffende.

Die Art und Weise der Aktivierungsfunktion für unterschiedliche chirurgische Handhabungselemente wird zum Teil konstruktiv sehr unterschiedlich ausgeführt, mit dem Ergebnis, dass die Aufrechterhaltung der sicherheitstechnischen Anforderungen entsprechend definierter Lebensdauer ebenso sehr unterschiedlich ausfallen kann.

Die bekannten Lösungen, mit denen das Eindringen von Feuchtigkeit bei Aufbereitung, Waschen oder Sterilisieren der Instrumente bzw. Handhabungsteile verhindert werden soll, weisen bestimmte systembedingte Nachteile auf:
Die mechanische Abdichtung der vielen Schnittstellen ist schwer realisierbar, insbesondere durch Materialveränderungen, induziert durch chemische/thermische Wiederaufbereitung. Bedingt durch das "dichte System" und eingeschlossene Luft (in großen Hohlräumen) findet durch Temperaturwechselbeanspruchung und entsprechenden Gasaustausch im Inneren der Griffe (initiiert durch Wasch- und Sterilisationsvorgänge) eine starke Beanspruchung der Dichtungselemente statt. Durch das Eindringen von Feuchtigkeit kann es zur Korrosion von metallischen Teilen (insbesondere Leiterbahnen, Kontakten, Verbindungen elektronischer Bauteile) kommen, die zu einem Funktionsausfall, im "Worst Case" zu einer Eigenaktivierung, führen können. Durch das Eindringen von Feuchtigkeit kann es speziell bei HF-führenden Strukturen zur Korrosion, initiiert durch elektrochemische Prozesse kommen, die funktionell und sicherheitstechnisch höchst bedenkliche Folgen haben kann.

Der Erfindung liegt die Aufgabe zugrunde, diese Probleme bekannter Handhabungsteile durch ein neues Konzept zu lösen, welches insbesondere die Bereitstellung von Handhabungsteilen mit uneingeschränkter Funktionalität und Erfüllung aller Sicherheitsanforderungen über eine geforderte Lebensdauer ermöglicht und zugleich Verbesserungen hinsichtlich der Herstellbarkeit und Prozesssicherheit der Handhabungsteile erbringt.

Diese Aufgabe wird gemäß einem ersten Aspekt der Erfindung durch die Bereitstellung einer neuartigen Schaltsteuereinrichtung für medizinische Instrumente mit den Merkmalen des Anspruchs 1 und gemäß einem zweiten Aspekt der Erfindung durch die Bereitstellung eines Handhabungsteils gemäß Anspruch 8 gelöst. Bereitgestellt wird zudem ein Satz medizinischer Instrumente mit entsprechenden Handhabungsteilen. Ausgestaltungen der Erfindung sind Gegenstand der abhängigen Ansprüche.

Die Erfindung schließt den Gedanken einer modularen Realisierung der eigentlichen Handhabungsfunktion und der Schaltsteuerfunktion(en) von Instrumenten der genannten Art ein. Diese Modularität sieht so aus, dass die Schaltsteuereinrichtung als relativ selbständiges, in sich räumlich geschlossenes Schaltsteuermodul mit dem entsprechenden Betätigungselement und Kommunikationsmitteln zum Zusammenwirken mit dem Instrument oder einem zugehörigen Gerät zur Realisierung der Schalt-/Steuerfunktion ausgebildet ist. Zur Erfindung gehört weiter der Gedanke, an diesem Schaltsteuermodul Fixierungsmittel zum lösbaren Anbringen in oder am Handhabungsteil vorzusehen. Schließlich ist es im Hinblick auf die vorgenannten Anforderungen und Probleme von Bedeutung, das Schaltsteuermodul in einer Weise druckdicht und hitzeunempfindlich auszuführen, dass es den beim Waschen, Sterilisieren etc. herrschenden Umgebungsbedingungen über viele Anwendungszyklen zuverlässig widersteht.

Das Schaltsteuermodul hat keine eigene elektrische Zuleitung, sondern greift mit seinen Kontaktmitteln in interne elektrische Verbindungen eines Instruments ein, welches über eine Instrumenten-Zuleitung mit dem Stromnetz verbindbar ist.

Ein wesentlicher Vorteil der vorgeschlagenen Lösung besteht darin, dass der Restgasanteil im Schaltsteuermodul aufgrund der geringen Baugröße sehr gering gehalten werden kann und die Beanspruchung der Teile (insbesondere des Gehäuses) aufgrund von Temperaturwechselbelastungen deutlich verringert werden kann. Die nach Entnehmen des Schaltsteuermoduls verbleibenden Handhabungsteile können undicht ausgeführt werden, d.h. die weiter oben erwähnte, konstruktiv und technologisch aufwendige Abdichtung von Schnittstellen ist nicht mehr notwendig. Insbesondere ergibt sich daraus eine einfache und prozesssichere Montagetechnik für die neuartigen, modular aufgebauten Handhabungsteile.

In bevorzugter Ausgestaltung der Erfindung kann das Schaltsteuermodul in einer "standardisierten" Bauform erforderlichenfalls in einer Vielzahl unterschiedlicher, aber systemkonformer Handhabungsteile bzw. Instrumente - und ggf. auch als selbständige Einheit, ohne räumlichen Bezug zu einem Handhabungsteil - eingesetzt werden. Dies ermöglicht weitere Vereinfachungen der Entwurfs- und Herstellungsprozesse sowie der Logistik in der Klinik bzw. Praxis.

In einer aus derzeitiger Sicht breite Anwendungsmöglichkeiten bietenden Ausführung ist vorgesehen, dass die Modul-Kontaktmittel Steckkontaktelemente, insbesondere Kontaktstifte oder einen Stecker, zum Eingriff in Anschlusskontakte des Handhabungsteils aufweisen. Alternativ hierzu kann vorgesehen sein, dass die Modul-Kontaktmittel eine drahtlose Sendeeinheit, insbesondere Bluetooth-, IR-oder Ultraschall-Sendeeinheit, aufweisen. Speziell die letztgenannte Ausführung ermöglicht auch eine Stand-alone-Funktion der Schaltsteuereinrichtung, räumlich losgelöst von einem Handhabungsteil im engeren Sinne.

In einer weiteren Ausführung der Erfindung ist vorgesehen, dass die Fixierungsmittel Rastmittel aufweisen, die insbesondere an das Gehäuse angeformt sind. Alternativ hierzu oder auch in Kombination hiermit können die Fixierungsmittel einen Schraubabschnitt aufweisen, der insbesondere an das Gehäuse angeformt ist. Auch weitere einfache Fixierungs- bzw. Verbindungsmittel an sich bekannter Art sind für die Herstellung einer lösbaren Verbindung zwischen Schaltsteuermodul und Handhabungsteil einsetzbar, so etwa Mittel in Art eines Bajonettverschlusses, Gleitführungen etc.

In besonders zweckmäßiger Ausführung kann das Betätigungselement durch einen verformbaren Abschnitt des Gehäuses gebildet sein. So kann etwa bei einer Schaltsteuereinrichtung, die einen Taster als Aktivierungselement aufweist, eine ins Gehäuse des Tasters, der zugleich das Gehäuse des Schaltsteuermoduls bildet, eingeformte Schaltfeder das Betätigungselement bilden.

Zweckmäßige Merkmale des erfindungsgemäßen Handhabungsteils ergeben sich bereits aus den obigen Ausführungen zur Ausgestaltung des Schaltsteuermoduls und werden daher hier nicht wiederholt. Insgesamt versteht es sich, dass handhaben-seitige Fixierungs- bzw. Verbindungsmittel sowie Kontaktmittel korrespondierend zu den entsprechenden Fixierungs- oder Kontaktmitteln des Schaltsteuermoduls ausgebildet sein werden. So weit funktional möglich, sind auch Merkmals-Vertauschungen möglich, so etwa das Vorsehen von Kontaktstiften in der Handhabe und von entsprechenden Kontaktbuchsen am Schaltsteuermodul.

Vorteile und Zweckmäßigkeiten der Erfindung ergeben sich im übrigen aus der nachfolgenden Beschreibung von Ausführungsbeispielen und -aspekten der Erfindung anhand der Figuren. Von diesen zeigen:
Fig. 1A bis 1C perspektivische Ansichten eines erfindungsgemäßen Handhabungsteiles mit herausnehmbarem Schaltsteuermodul,
Fig. 2 eine Längsschnittdarstellung eines Tasters als Bestandteil des in Fig. 1A bis 1C gezeigten Schaltsteuermoduls,
Fig. 3 eine skizzenartige Darstellung einer weiteren Ausführungsform der Erfindung und
Fig. 4 eine skizzenartige Darstellung eines erfindungsgemäß ausgestalteten Satzes medizinischer Instrumente.

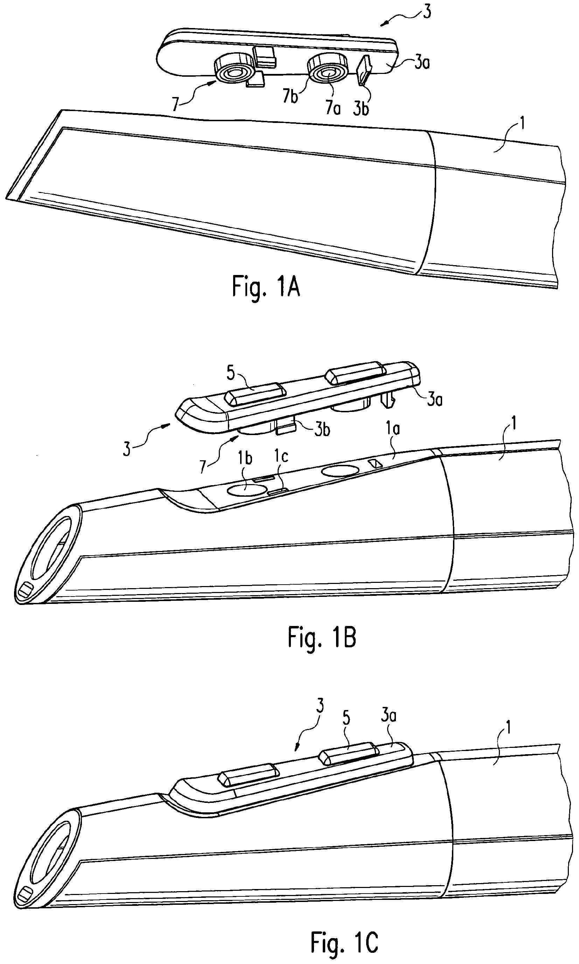

Fig. 1A bis 1C zeigen einen HF-Elektroden-Handgriff 1 zum Führen einer (nicht dargestellten) HF-Elektrode für die HF-Chirurgie, der ein herausnehmbares Schaltsteuermodul 3 enthält. Das Schaltsteuermodul 3 hat ein Gehäuse 3a mit angeformten Rasthaken 3b und zwei Drucktasten 5 als Betätigungselemente für je einen in das Gehäuse 3a eingesetzten Ein-/Aus-Schalter 7. Die Schalter dienen zur Umschaltung zwischen verschiedenen (elektrischen) Betriebsarten des Instruments. Anstelle eines oder beider Schalter können auch Dreh- oder Schiebesteller zur Steuerung elektrischer Betriebsparameter des Instruments vorgesehen sein.
Jeder Schalter 7 hat ringförmig ausgeführte Innen- und Außenkontakte 7a, 7b. Am Handhabungsteil 1 ist ein angeschrägter Oberflächenabschnitt 1a zum Aufsetzen des Schaltsteuermoduls 3 vorgesehen, und in diesem gibt es erste Ausnehmungen 1b, die die Schalter 7 aufnehmen können, und zweite Ausnehmungen 1c, die als handhaben-seitige Fixierungs- bzw. Rastmittel mit den Rasthaken 3b am Schaltsteuermodul zusammenwirken.

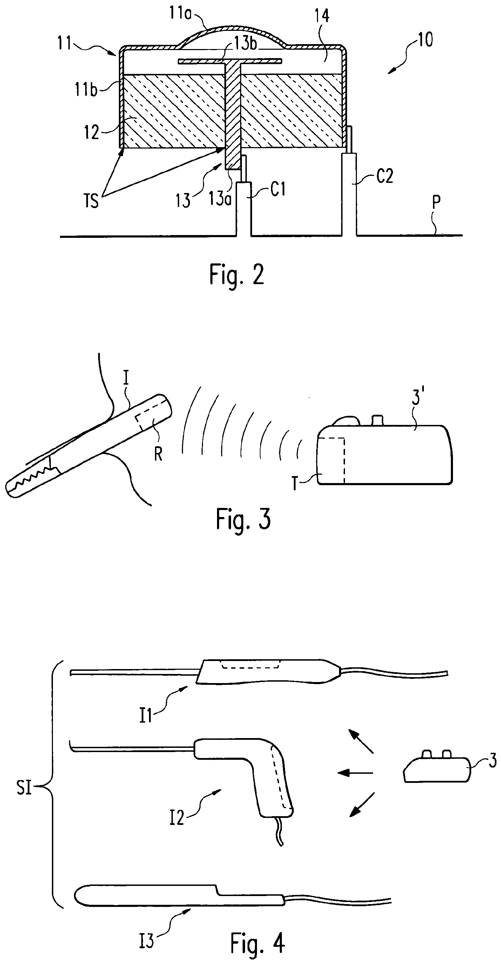

Fig. 2 zeigt einen Taster 10, verbunden mit zwei äußeren Anschlusskontakten C1 und C2, die auf einer Platine P eines medizinischen Handhabungsteils angeordnet sind. Der Taster 10, der in der Fig. 1A bis 1C dargestellten Ausführung der Erfindung anstelle des Schalters 7 eingesetzt sein kann, besteht aus einem ersten Gehäuseteil 11 in Form eines Topfes mit kugelabschnittförmig ausgebeultem Boden 11a und einer zylindrischen Wandung 11b, einem ringförmigen zweiten Gehäuseteil 12 aus Glas und einem in das Glas-Gehäuseteil 12 zentrische eingesetzten Kontaktstück (Gegenkontakt) 13. Das zweite Gehäuseteil ist in seinem Außendurchmesser so bemessen, dass es praktisch spielfrei in die zylindrische Wandung 11b des ersten Gehäuseteils 11 passt, und in die zylindrische Durchführung in seinem Zentrum fast ebenso spielfrei ein Stiftabschnitt 13a des Kontaktstücks 13.
Durch einen thermischen Bearbeitungsschritt ist an den Berührungsflächen der genannten Teile jeweils eine hermetisch dichte Versiegelung TS geschaffen. Das Kontaktstück 13 ist im Taster 10 so platziert, dass eine kreisförmige Kontaktplatte 13b an seinem inneren Ende in einem Hohlraum 14 zwischen der innenseitigen Stirnfläche des zweiten Gehäuseteils 12 und dem Boden des ersten Gehäuseteils 11 zu liegen kommt, und zwar mit solchem Abstand unterhalb der Beule 11a im Boden des ersten Gehäuseteils, das diese beim federnden Niederdrücken die Kontaktplatte 13b berührt. Hierdurch wird eine (temporäre) elektrische Verbindung zwischen dem Anschlusskontakt C1, an den das Kontaktstück 13 angeschlossen ist, und dem Anschlusskontakt C2 hergestellt, an den das leitfähige erste Gehäuseteil 11 angeschlossen ist. Die Beule 11a dient somit als Schaltfeder des Tasters 10 und kann in einer Abwandlung der Ausführung nach Fig. 1A bis 1C unmittelbar die Betätigungstaste ersetzten oder aber unter dieser angeordnet sein.

Fig. 3 zeigt skizzenartig als weitere Ausführung der Erfindung ein Schaltsteuermodul 3', welches (in einer anderen Konfiguration) mit einem Handhabungsteil eines medizinischen Instruments verbunden werden kann, in der dargestellten Konfiguration aber nicht in eine Handhabe integriert ist, sondern vom Bediener eigenständig gehandhabt wird, um Steuerfunktionen an einem endoskopischen Instrument I auszuführen. Die Signalverbindung erfolgt über eine Bluetooth-Funkstrecke, und hierzu weist das Schaltsteuermodul 3' eine Bluetooth-Funksendeeinheit T und das Instrument I eine entsprechende Empfangseinheit R auf.

Fig. 4 zeigt skizzenartig einen Satz SI medizinischer Instrumente I1 bis I3 mit unterschiedlich gestalteten Handhabungsteilen 1.1 bis 1.3, welche jeweils eine feste Anschlussleitung und einheitlich gestaltete Aufnahmen für ein Schaltsteuermodul 3 aufweisen. Jedes der Instrumente kann somit mit der gleichen Schaltsteuereinheit bestückt werden bzw. es ist nur ein einziger Typ von Schaltsteuereinrichtungen für alle Instrumente herzustellen und am Lager zu halten.

Die Ausführung der Erfindung ist nicht auf die oben angeführten Beispiele und hervorgehobenen Aspekte beschränkt, sondern ebenso in einer Vielzahl von Abwandlungen möglich, die im Rahmen fachgemäßen Handelns liegen.

## Patentansprüche

1. Schaltsteuereinrichtung eines eine Anschlussleitung aufweisenden medizinischen Instruments, insbesondere einer Elektrode für die HF-Chirurgie, ausgebildet als Schaltsteuermodul (3) zur Steuerung verschiedener elektrischer Betriebsarten des Instruments mit einem räumlich und druckdicht geschlossenen, gegenüber Sterilisationstemperaturen beständigen Gehäuse (3a), einem Betätigungselement (5) und Fixierungsmitteln (3b) zum lösbaren Anbringen in oder an einem Handhabungsteil (1) des Instruments sowie Modul-Kontaktmitteln (7a, 7b) zum Zusammenwirken mit Geräte- oder Instrumenten-Kontaktmitteln, die ihrerseits mit der Anschlussleitung verbunden sind, zur Ausführung einer Schaltsteuerfunktion.

2. Schaltsteuereinrichtung nach Anspruch 1, wobei die Modul-Kontaktmittel Steckkontaktelemente, insbesondere Kontaktstifte oder einen Stecker, zum Eingriff in Anschlusskontakte des Handhabungsteils aufweisen.

3. Schaltsteuereinrichtung nach Anspruch 1, wobei die Modul-Kontaktmittel eine drahtlose Sendeeinheit insbesondere Bluetooth-, IR- oder Ultraschall-Sendeeinheit, aufweisen.

4. Schaltsteuereinrichtung nach einem der vorangehenden Ansprüche, wobei die Fixierungsmittel Rastmittel aufweisen, die insbesondere an das Gehäuse angeformt sind.

5. Schaltsteuereinrichtung nach einem der vorangehenden Ansprüche, wobei die Fixierungsmittel einen Schraubabschnitt aufweisen, der insbesondere an das Gehäuse angeformt ist.

6. Schaltsteuereinrichtung nach einem der vorangehenden Ansprüche, die einen Taster aufweist.

7. Schaltsteuereinrichtung nach einem der vorangehenden Ansprüche, wobei das Betätigungselement durch einen druck-verformbaren Abschnitt des Gehäuses gebildet ist.

8. Handhabungsteil (1) eines medizinischen Instruments, mit einer Ausnehmung (1b) und Handhaben-Befestigungsmitteln (1c) sowie Handhaben-Kontaktmitteln zum lösbaren Anbringen und steuersignalmäßigen Anschluss einer Schaltsteuereinrichtung nach einem der vorangehenden Ansprüche.

9. Handhabungsteil nach Anspruch 8, wobei die Handhaben-Kontaktmittel Anschlusskontakte zum Eingriff von Steckkontaktelementen der Schaltsteuereinrichtung aufweisen.

10. Handhabungsteil nach Anspruch 8, mit einer drahtlosen Empfangseinheit, insbesondere Bluetooth-, IR- oder Ultraschall-Empfangseinheit, zum Zusammenwirken mit einer entsprechenden drahtlosen Sendeeinheit der Schaltsteuereinrichtung.

11. Handhabungsteil nach einem der Ansprüche 8 bis 10, mit Rastmitteln und/oder einem Schraubabschnitt, die zu entsprechenden Fixierungsmitteln der Schaltsteuereinrichtung korrespondierend ausgeführt sind.

12. Satz medizinischer Instrumente mit Handhabungsteilen nach einem der Ansprüche 8 bis 11, wobei die Handhabungsteile aller Instrumente gleichartige Ausnehmungen, Handhaben-Befestigungsmittel und Handhaben-Kontaktmittel zum Anbringen und steuersignalmäßigen Anschluss ein und derselben Schaltsteuereinrichtung aufweisen.

## Claims

1. A switching control device for a medical instrument comprising a connection line, in particular for an electrode for HF surgery, designed as a switching control module (3) for controlling various electrical operating modes of the instrument and having a three-dimensionally and pressure-tightly sealed housing (3a) that is resistant to sterilization temperatures, an actuating element (5) and fixing means (3b) for releasably mounting within or on a manipulating part (1) of the instrument, and module contacting means (7a, 7b) for interacting with device or instrument contacting means, which in turn are connected to the connection line to perform a switching control function.

2. The switching control device according to claim 1, wherein the module contacting means comprise plug contact elements, in particular contact pins or a plug for engagement in connection contacts of the manipulating part.

3. The switching control device according to claim 1, wherein the module contacting means comprise a wireless transmission unit, in particular a Bluetooth, IR or ultrasound transmission unit.

4. The switching control device according to anyone of the preceding claims, wherein the fixing means comprise latching means which are in particular integrally molded with the housing.

5. The switching control device according to anyone of the preceding claims, wherein the fixing means comprise a screw portion which is in particular integrally molded with the housing.

6. The switching control device according to anyone of the preceding claims, comprising a push button.

7. The switching control device according to anyone of the preceding claims, wherein the actuating element is formed by a pressure-deformable portion of the housing.

8. A manipulating part (1) of a medical instrument, having a recess (1b) and handle fixing means (1c) as well as handle contacting means for releasably mounting and connecting a switching control device according to anyone of the preceding claims in a manner dependent on a control signal.

9. The manipulating part according to claim 8, wherein the handle contacting means comprise connection contacts for the engagement of plug contact elements of the switching control device.

10. The manipulating part according to claim 8, having a wireless reception unit, in particular a Bluetooth, IR or ultrasound reception unit, for interacting with a corresponding wireless transmission unit of the switching control device.

11. The manipulating part according to anyone of claims 8 to 10, having latching means and/or a screw portion realized to be corresponding to respective fixing means of the switching control device.

12. A kit of medical instruments with manipulating parts according to anyone of claims 8 to 11, wherein the manipulating parts of all of the instruments comprise recesses, handle fixing means and handle contacting means of the same kind for mounting and connecting one and the same switching control device in a manner dependent on a control signal.

## Revendications

1. Dispositif de commande de commutation d'un instrument médical présentant une ligne de raccordement, en particulier d'une électrode pour la chirurgie haute fréquence, constitué comme module de commande de commutation (3) pour la commande de divers modes de fonctionnement électriques de l'instrument, comportant un boîtier (3a) spatialement fermé de manière étanche à la pression et résistant aux températures de stérilisation, un élément d'actionnement (5) et des moyens de blocage (3b) pour la mise en place amovible dans ou sur une pièce de manipulation (1) de l'instrument ainsi que des moyens de contact de module (7a, 7b) pour l'interaction avec des moyens de contact d'appareil ou d'instrument, lesquels sont de leur côté reliés à la ligne de raccordement, pour l'exécution d'une fonction de commande de commutation.

2. Dispositif de commande de commutation selon la revendication 1, sachant que les moyens de contact de module présentent des éléments de contact enfichables, en particulier des broches de contact ou une fiche, pour la mise en prise dans des contacts de raccordement de la pièce de manipulation.

3. Dispositif de commande de commutation selon la revendication 1, sachant que les moyens de contact de module présentent une unité d'émission sans fil, en particulier une unité d'émission à Bluetooth, IR ou ultrasons.

4. Dispositif de commande de commutation selon l'une des revendications précédentes, sachant que les moyens de blocage présentent des moyens d'encliquetage qui sont en particulier intégralement moulés au boîtier.

5. Dispositif de commande de commutation selon l'une des revendications précédentes, sachant que les moyens de blocage présentent une section filetée qui est en particulier intégralement moulée au boîtier.

6. Dispositif de commande de commutation selon l'une des revendications précédentes, lequel présente un bouton-poussoir.

7. Dispositif de commande de commutation selon l'une des revendications précédentes, sachant que l'élément d'actionnement est formé par une section du boîtier qui est déformable par pression.

8. Pièce de manipulation (1) d'un instrument médical, comportant un évidement (1b) et des moyens de fixation de manipulation (1c) ainsi que des moyens de contact de manipulation pour la mise en place amovible et le raccordement en termes de signal de commande d'un dispositif de commande de commutation selon l'une des revendications précédentes.

9. Pièce de manipulation selon la revendication 8, sachant que les moyens de contact de manipulation présentent des contacts de raccordement pour la mise en prise d'éléments de contact enfichables du dispositif de commande de commutation.

10. Pièce de manipulation selon la revendication 8, comportant une unité de réception sans fil, en particulier une unité de réception à Bluetooth, IR ou ultrasons, pour l'interaction avec une unité d'émission sans fil correspondante du dispositif de commande de commutation.

11. Pièce de manipulation selon l'une des revendications 8 à 10, comportant des moyens d'encliquetage et/ou une section filetée qui sont exécutés de manière correspondante à des moyens de blocage correspondants du dispositif de commande de commutation.

12. Jeu d'instruments médicaux comportant des pièces de manipulation selon l'une des revendications 8 à 11, sachant que les pièces de manipulation de tous les instruments présentent des évidements, des moyens de fixation de manipulation et des moyens de contact de manipulation de même type pour la mise en place et le raccordement en termes de signal de commande d'un seul et même dispositif de commande de commutation.
